# FASCICULE DE BREVET EUROPEEN

(11) **EP 3 691 567 B1**
(45) Date de publication et mention de la délivrance du brevet: **28.07.2021**
(21) Numéro de dépôt: 18797004.1
(22) Date de dépôt: 02.10.2018
(51) Int. Cl.: A61F 2/06, A61F 2/24, D04B 23/00, A61F 2/00

(54) **INSERT TEXTILE A FINALITÉ MÉDICALE ET SON PROCÉDÉ DE FABRICATION**
TEXTILEINLAGE FÜR MEDIZINISCHE ZWECKE UND VERFAHREN ZU IHRER HERSTELLUNG
TEXTILE INSERT FOR MEDICAL PURPOSES, AND METHOD FOR PRODUCING SAME

(30) Priorité: 04.10.2017 FR 1759264
(43) Date de publication de la demande: 12.08.2020
(73) Titulaire: MDB Texinov, 38110 Saint Didier de la Tour (FR); Deltaval, 69006 Lyon (FR)
(72) Inventeur: MIGNOT, Eric, 38620 Velanne (FR); LEO, Sébastien, 38850 Bilieu (FR); PEYRACHON, Maud, 38500 Voiron (FR); BOUCARD, Nadège, 38480 Pont De Beauvoisin (FR); TANKERE, Jacques, 01800 Meximieux (FR)
(74) Mandataire: Cabinet Laurent & Charras
(86) Numéro de dépôt international: PCT/FR2018/052428
(87) Numéro de publication internationale: WO 2019/069014

(56) Documents cités:
- EP-A1- 0 338 994
- WO-A1-2014/149612
- FR-A1- 3 041 663
- US-A- 2 978 787

## Description

### DOMAINE DE L'INVENTION

L'invention s'inscrit dans le domaine des inserts textiles, notamment à usage médical. Elle concerne tant les implants proprement dits que des structures textiles destinées à recouvrir des implants de nature plus conventionnelle.

La pose d'une prothèse ou d'un implant s'accompagne d'une réaction inflammatoire, celle-ci étant d'autant plus aigüe que la tolérance biologique de l'individu est faible. La formation d'un bon tissu cicatriciel nécessite ensuite une intense activité fibroblastique.

La phase d'activité fibroblastique est précédée d'une période critique durant laquelle la stabilité de l'implant doit être assurée par des points de fixation. L'implant donc doit permettre une bonne adhérence aux tissus, de telle sorte à favoriser le développement d'une forte colonisation cellulaire qui accompagne la formation d'un tissu cicatriciel de qualité.

### ART ANTERIEUR

Les implants actuellement utilisés sont généralement confectionnés à partir, soit de matériaux métalliques, tels que l'acier inoxydable, le titane et ses alliages, soit de matériaux plastiques. Si ces implants ont alors d'excellentes propriétés mécaniques, la faible adhérence des tissus constitue un frein majeur à leur mise en œuvre. En effet, en raison de cette faible adhérence, la colonisation cellulaire de l'implant et donc corollairement la formation d'un tissu cicatriciel de qualité sont défaillantes. Par ailleurs, dans le cas d'implants métalliques, le risque de toxicité dû à l'accumulation d'ions métalliques suite à la corrosion n'est pas exempt.

Afin de pallier cette difficulté, diverses techniques ont été développées, visant à favoriser l'intégration des implants de type métallique ou plastique. Parmi celles-ci, certaines consistent à greffer ou à enduire des produits chimiques à la surface de l'implant en question. Ces produits induisent la formation de structures microporeuses à la surface de l'implant, tel que par exemple décrit dans le document WO 2014/024171. Les exemples de traitement d'implants par ensemencement, dépôt de vapeur, électrolyse et dépôt de divers produits chimiques sont très nombreux. Certaines techniques préconisent par ailleurs la création de microporosités en surface de l'implant par attaque chimique, comme décrit dans le document EP 0.388.576.

Ces techniques de traitement de surface de l'implant nécessitent cependant une étude systématique de la compatibilité entre la couche déposée et le matériau de l'implant, qu'il soit métallique ou réalisé en matière plastique. On observe en effet souvent une unicité du couple matériau déposé / implant, de laquelle dépendent les risques de relargage du matériau déposé, relargages de plus en plus encadrés par les normes en vigueur, ces dernières tendant même vers un risque zéro de relargage.

En outre, ces techniques peuvent s'avérer coûteuses et complexes à mettre en œuvre, la méthode de revêtement choisie ne s'adaptant pas nécessairement aux formes parfois complexes de l'implant concerné.

On a également proposé de remplacer les implants métalliques par des implants céramiques. On a en effet pu constater avec de tels implants céramiques, la création de micro et nano porosités par oxydation de la surface, conduisant à une bonne adhérence cellulaire, tel que par exemple décrit dans le document US 2010/274361.

Ces procédés sont néanmoins longs et coûteux, et les performances des implants en céramique, bien qu'en constante évolution, ne sont pas encore au niveau de celles des implants métalliques ou plastiques.

Une autre approche consiste à utiliser des implants textiles, soit en tant que tel, soit en association avec les matériaux précédemment évoquées de façon à les recouvrir ou à assurer une liaison avec les tissus biologiques facilitant éventuellement la fixation par le chirurgien. Ceux-ci présentent de bonnes capacités de colonisation, celles-ci étant favorisées d'une part, par les matériaux utilisés pour leur fabrication (PET, polypropylène, ...), d'autre part par leur structure faite de fils et enfin par le calibrage de leur maillage, définissant des pores, la taille desdits pores étant déterminante tant pour la rigidité que pour l'accrochage des tissus cicatriciels sur l'implant.

Les parties textiles de ces implants sont réalisés soit en structure tissée, soit en structure tricotée. Elles nécessitent souvent une phase de découpe et d'assemblage faite à la main, qui s'avère couteuse, et dont la reproductibilité est délicate puisque liée à l'opérateur. L'objectif visé par la présente invention est de proposer un insert implantable sous la forme de textile micro-tubulaire. L'invention consiste donc en la réalisation d'inserts, sous la forme de multi micro-tubes, permettant une faible inflammation accompagnée d'une forte activité fibroblastique.

Cet insert est obtenu en une seule étape, présente une capacité accrue à être colonisé, tout en permettant une meilleure maniabilité de l'insert par le chirurgien et donc une utilisation plus simple. L'invention permet ainsi de supprimer ou de réduire la phase de confection manuelle, ce qui aboutit à une meilleure reproductibilité et réduit les coûts.

US2978787 A divulgue le préambule de la revendication 1.

### EXPOSE DE L'INVENTION

Aussi l'invention vise-t-elle la réalisation d'un insert textile tricoté en trois dimensions conformément aux revendications.

Cet insert se présente sous la forme d'un multi-micro tubes pouvant servir soit à recouvrir des implants tubulaires, ou de forme complexe et corollairement favoriser la colonisation autour dudit implant, ou à favoriser la fixation de l'implant sur le tissu biologique par le chirurgien, soit à être utilisé directement en tant qu'implant.

Cet insert textile tricoté en trois dimensions est obtenu en une seule étape par la technologie à maille jetée, sur un métier Rachel à double fonture ou sur métier crochet. L'insert obtenu comporte, dans le sens production au moins deux tubes parallèles entre eux, et séparés par une zone au niveau de laquelle les nappes résultant du tricotage sur les deux fontures sont liées entre elles. Ces au moins deux tubes définissent un tube principal et au moins un tube connexe.

Il est introduit dans au moins l'un des tubes connexes, directement sur le métier à tricoter, c'est-à-dire lors de la réalisation de l'insert, un fil droit ou un assemblage de fils montés en cabestan, donc qui ne maille pas, une bandelette, des fibres optiques, ou tout autre matériau dont la dimension principale est la longueur (c'est-à-dire la dimension principale de l'inscrt), une tige, un jonc, ou un élément rigidifiant, dont la finalité est de faciliter la mise en œuvre de l'implantation de l'insert.

L'utilisation d'un métier RACHEL à maille jetée à double fonture ou d'un métier crochet permet d'une part la fabrication simultanée de tubes parallèles ou sensiblement parallèles entre eux, et d'autre part la création de liages ou d'armures qui déterminent une structure de mailles comprenant une certaine ouverture ou porosité, paramètre important pour favoriser la colonisation ultérieure de l'implant.

Avantageusement, cette porosité peut varier d'un tube à l'autre en jouant sur la taille et la structure des fils mis en œuvre, ceux-ci pouvant être mono ou multi-filaments, mais également sur le débit desdits fils dans le métier.

L'ouverture des pores peut être comprise entre 0,05 et 3 millimètres dans sa plus grande dimension, afin de faciliter la colonisation et la tenue de l'implant lors de la pose par le chirurgien. La dimension de ces portes résulte du choix de l'armure, de la grosseur du fil et la densité de mailles.

Les au moins deux tubes, respectivement principal et connexe ainsi obtenus peuvent être de diamètres identiques ou différents.

Selon l'invention, l'un des micro-tubes, qu'il soit positionné ou non en bordure de l'insert, est ouvert. En d'autres termes, aucun fil de liage ne vient mailler pour définir le tube en question. Les nappes résultant des deux fontures du métier sont donc libres et sont susceptibles de constituer des zones de coutures de l'insert autour d'un autre élément, tel que par exemple un tube métallique en nickel, titane ou autre, un cathéter ou un ancillaire.

Selon l'invention, l'un des au moins deux tubes, et en l'espèce le tube principal, présente un diamètre nettement supérieur au diamètre de l'autre ou des autres tubes. Ce tube de plus grand diamètre a pour finalité de recevoir un cathéter, un tube de type Nitinol (alliage de nickel et de titane), permettant de fixer un cathéter ou un autre élément fonctionnel lors de l'intervention, ou un élément artificiel de remplacement d'un organe, tel que par exemple un anneau de prothèse valvulaire cardiaque.

Typiquement le diamètre du tube en question est d'au moins 3 millimètres. Il peut atteindre 15 millimètres, voire être plus important en fonction des applications envisagées. Le diamètre des tubes latéraux est, comme déjà dit plus réduit, et typiquement compris entre 0,5 et 2 millimètres. On détermine le diamètre des tubes par le choix de la jauge du métier, l'enfilage sur chaque barre dudit métier et par le nombre d'aiguilles au travail.

Selon l'invention, les tubes constitutifs de l'insert sont soit tangents entre eux, soit séparés l'un de l'autre ou les uns des autres d'une zone plate résultant du liage des nappes issues de chaque fonture ou de l'absence de fil dans l'une des deux fontures.

Selon une forme de réalisation de l'invention, l'insert comporte trois tubes, respectivement un tube central principal d'un diamètre nominal d'au moins 3 millimètres, du type de celui évoqué précédemment, tandis que les autres tubes sont de diamètres inférieurs, et typiquement compris entre 0,5 et 2 millimètres. Dans le cas où le multi-micro tube est constitué de trois tubes, il y a alors un tube central principal sur lequel viennent s'ajouter deux tubes plus petits dits tubes connexes, positionnés de façon diamétralement opposée de part et d'autre du tube central.

La technique mise en œuvre pour la réalisation de l'insert de l'invention permet d'introduire directement lors de la fabrication de l'insert, dans le sens production, des tiges ou joncs, et de manière générale des éléments rigidifiant, qui se retrouvent dans les tubes latéraux de diamètre inférieur. Ce faisant, l'introduction de l'insert ainsi réalisé au niveau de son lieu d'implantation est facilitée. Corollairement, la réalisation d'une zone de suture est favorisée.

Comme évoqué ci-dessus, l'invention permet d'introduire en une seule étape, dans les tubes de l'insert (préférentiellement les tubes connexes c'est-à-dire ceux situés aux bords), un fil droit ou un assemblage de fils qui ne maillent pas (en cabestan), une bandelette en polymère ou Nitinol par exemple, une ou des fibres optiques, ou tout autre matériau dont la dimension principale est la longueur.

Les tubes connexes ou latéraux sont réalisés préférentiellement avec la même armure que celle du tube central, la géométrie de l'insert textile étant par conséquent constante et permettant donc une colonisation homogène.

Selon la complexité de cette armure, 6 à 16 barres du métier à maille jetée à double fonture sont utilisées pour réaliser l'invention, permettant ainsi de faire varier le nombre de tubes et leur diamètre respectif.

Selon une forme de réalisation de l'invention, la zone de liaison entre les différents tubes est étroite (1 à 2 colonnes de mailles). Cette zone de liaison est réalisée dans la même armure que les autres parties des tubes afin d'assurer une porosité du textile constante et donc une colonisation homogène.

Alternativement, la réalisation d'une zone de connexion inter-tubes est plate et d'une largeur allant de 5 millimètres à plusieurs centimètres. Ce faisant, il est possible avec l'insert de l'invention de s'adapter aux besoins du praticien.

Selon un autre mode de réalisation, les nappes résultant du tricotage sont liées entre elles au moins sur l'un de leurs bords dans le sens production. Il y a alors formation d'ailettes qui servent de support de suture ou d'assemblage de l'insert avec d'autres éléments du dispositif médical final. La largeur des ailettes ainsi formées peut varier en fonction du liage. Corollairement, l'assemblage de plusieurs inserts de l'invention entre eux peut également être réalisé par le biais des ailettes ainsi définies, notamment par couture.

La présente invention peut également voir sa structure renforcée localement, soit par le jeu d'armure ou des barres du métier à tricoter, soit en modifiant localement la densité des mailles par pilotage électronique des barres et densité de mailles qui peuvent ainsi varier.

La zone de renfort ainsi créé peut demeurer indentifiable grâce à l'utilisation de fils de couleur. Un tel renfort peut ainsi être localisé, notamment au niveau de la ou des zones soumises à un effort lorsque l'insert est implanté. Il peut également servir pour optimiser l'agrafage ou la suture de l'insert une fois implanté.

Selon les fonctions dévolues à l'insert implantable, un ou plusieurs des tubes peuvent être pleins, c'est-à-dire que le volume défini par le ou les tubes en question est rempli par des fils dits « de poil ». Ces fils sont les fils usuels de liaison répartis sur une seule ou sur plusieurs barres d'un métier double fonture. Ces fils passent alternativement par le mouvement du métier d'une fonture à l'autre pour créer un textile présentant une épaisseur.

Les différentes propriétés mécaniques sont obtenues par le choix de l'armure.

En outre, l'élasticité d'allongement de l'insert dans le sens travers ou dans le sens production est possible et peut être réglable par le choix de l'armure, et ce pour des degrés variables par le choix des armures bien connues, telles que par exemple chainette, chainette tramée, satin.

Par ailleurs, le choix de l'armure rend le textile constitutif de l'insert indémaillable.

Selon l'invention, l'insert textile tricoté est réalisé en polymère d'origine naturelle résorbable ou non, ou en polymère ou copolymère synthétique, résorbable ou non, les matériaux pouvant être les suivants : polypropylène, polyéthylène, polyester, polyamides, bio-polyesters (PLA : acide polylactique, PGA : acide polyglycolique, PCL : polycaprolactone, PLGA: acide poly lactique-co-glycolique), PDO : polydioxanone, Vectran® (polyester aromatique), aramides, fibres minérales, fils gainés...

Les fils mis en œuvre dans cet insert textile tricoté peuvent être mono-filaments de diamètre compris entre 30 et 500 micromètres, ou des multi-filaments dont le titre varie entre 10 et 5000 Dtex.

Par ailleurs, d'autres fils sont susceptibles d'être introduits dans l'insert de l'invention lors de la réalisation de ce dernier, afin de lui conférer des propriétés particulières. Typiquement, ces fils peuvent être métalliques (par exemple en cuivre, en Nickel, en Argent, en Or, en Aluminium) ou à mémoire de forme de type Nitinol ou en polymère (par exemple PET (polyéthertéréphtalate, polypropylène, polyéthylène, polytétrafluoroethylène, e-PTFE, PLA, PGA, PCL, PDO, aramides.

Les avantages résultant d'un tel insert à micro tubes sont nombreux. En premier lieu, il convient de souligner qu'un tel insert est quasiment finalisé en sortie du métier à tricoter, limitant dès lors la ou les phases de confection manuelle. Ce faisant, d'une part, on en limite le coût de production, et d'autre part, on en rend la reproductibilité plus fiable.

Par ailleurs, la manipulation d'un tel insert par le praticien est facilitée, ledit insert étant rendu plus maniable grâce à une capacité de préhension accrue. La réalisation de l'intervention est également facilitée et se fait avec des risques amoindris, la fixation par des points de suture ou des agrafes du dispositif recouvert de textile étant plus simple pour le praticien.

En outre, sa porosité lui garantit une capacité accrue à être colonisé, facilitant de ce fait l'intégration de l'insert dans le corps humain.

Enfin, sa forme élaborée limite les opérations ultérieures de confection autour de l'implant, constituant un facteur de sécurité et de réduction des coûts supplémentaire.

### DESCRIPTION SOMMAIRE DES FIGURES

La manière dont l'invention peut être réalisée et les avantages qui en découlent, ressortiront mieux des exemples de réalisation qui suivent, donnés à titre indicatif et non limitatif, à l'appui des figures annexées.
La figure 1 est une représentation schématique d'un insert conforme à l'invention, constitué d'un triple micro tube.
La figure 2 est une représentation schématique de l'insert de la figure 1, dans lequel les tubes extérieurs reçoivent chacun un organe rigidifiant, tel qu'un jonc.
Les figures 3 et 4 sont des vues schématiques d'un autre mode de réalisation de l'invention, présentant cette fois-ci une ou plusieurs ailettes de part et d'autre du tube central.
Les figures 5 et 6 sont des représentations schématiques de variantes de l'insert de l'invention, présentant plusieurs tubes parallèles disposés soit de manière tangentielle ou quasi-tangentielle les uns par rapport aux autres, soit espacés par des parties plates.
La figure 7 est une représentation schématique d'une armure mise en œuvre pour la réalisation d'un multi-micro tube tel que représenté au sein dans les figures 1 et 2.
La figure 8 est une représentation schématique d'une armure mise en œuvre pour la réalisation d'un multi-micro tube, tel que représenté au sein de la figure 3.
La figure 9 est une représentation schématique d'une variante de la figure 2, présentant un tube central ouvert.

### DESCRIPTION DETAILLEE DE L'INVENTION

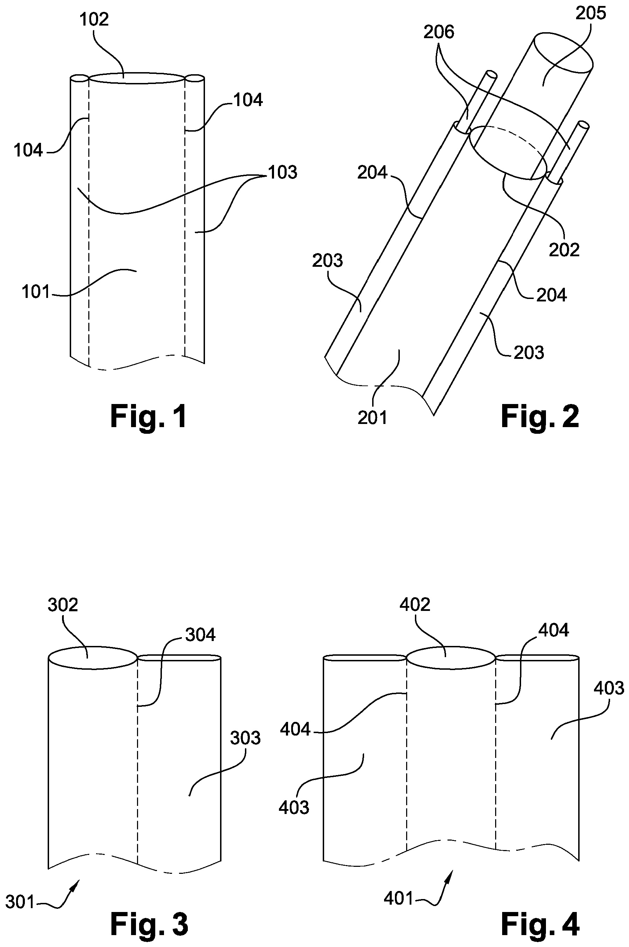

Les figures 1 et 2 sont, comme déjà indiqué, des vues schématiques d'un triple micro-tube. Ce multi-micro tube (101) est réalisé sur métier à maille jetée à double fonture, typiquement un métier RACHEL, et présente une structure en trois dimensions.

Il est ainsi réalisé simultanément sur ledit métier :
- un tube principal (102, 202), en l'espèce susceptible de présenter un diamètre de l'ordre de 3 à 4 millimètres ;
- deux micro-tubes (103, 203) connexes, disposés parallèlement au tube principal (102, 202), et diamétralement opposés l'un de l'autre par rapport audit tube principal (102, 202) ; ces deux tubes (103, 203) sont susceptibles de présenter un diamètre typique de l'ordre de 0,3 à 5 millimètres.

Le diamètre respectif des tubes (102, 202; 103, 203) dépend de la zone de liage (104, 204), c'est-à-dire de la distance entre deux zones de liage consécutives.

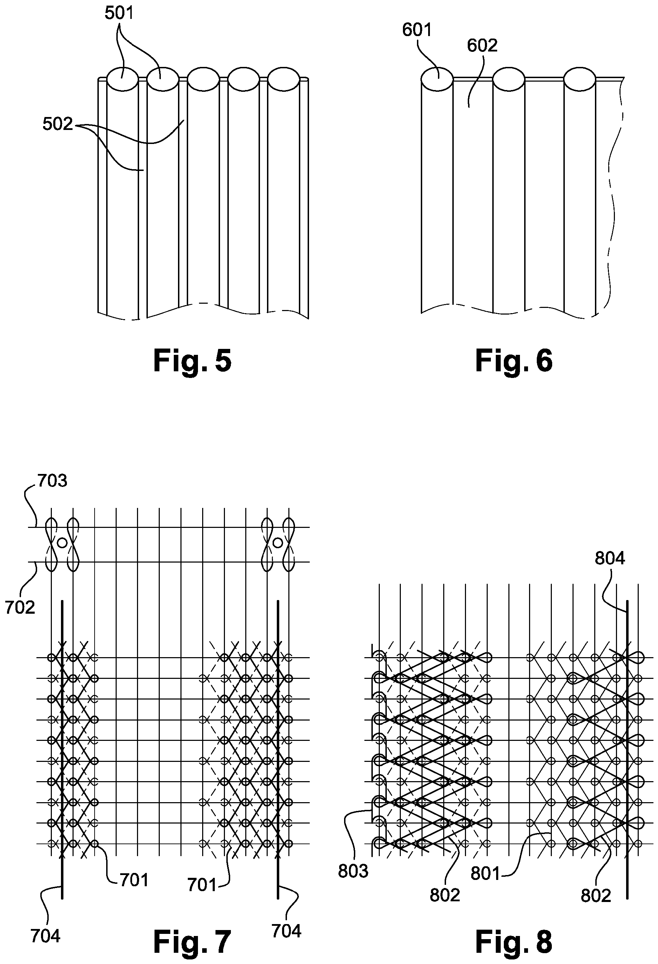

Corollairement, ces différents tubes s'étendant sens production, peuvent être soit tangents entre eux, tel qu'illustré au sein des figures 1 et 2, soit séparés les uns des autres d'une zone plate (502, 602) d'une distance plus ou moins grande - fonction des nécessités imposées pour la mise en œuvre de l'insert en question, et tel qu'illustré au sein des figures 5 et 6.

Le tube principal (102, 202) peut accueillir un cathéter, ou un tube de type Nitinol ou un élément artificiel de remplacement d'un organe donné de type anneau de valve cardiaque, comme illustré sur la figure 2 avec l'élément (205). Ce cathéter ou équivalent est inséré dans le tube (102, 202) après réalisation de l'insert sur le métier à tricoter. Cette insertion est rendue possible en raison de la rigidité de l'élément (205).

Cette même figure 2 illustre également l'introduction de joncs ou tiges ou de mono-fils spécifiques montés en cabestan (206) introduits directement lors de la fabrication sur le métier lorsque la rigidité est limitée. Ces tiges, joncs ou fils (206) permettent de faciliter l'introduction de l'insert dans le corps du patient. Ils définissent en outre une zone de suture plus aisée et sûre de fixation par le chirurgien.

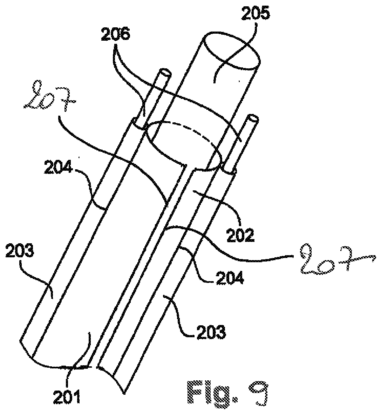

La figure 9 illustre une variante de la figure 2, dans laquelle le tube central ou principal (202) est ouvert. On a matérialisé par la référence (207) les bords ainsi définis par le tube ouvert. Afin d'obtenir un tel tube ouvert, on supprime des fils à certains endroits de l'une des deux fontures du métier. Alternativement, il est également possible d'obtenir un tel tube ouvert par adaptation de l'enfilade ou de l'armure comme pour la création de deux laizes su un métier simple fonture, technique parfaitement maitrisée par l'homme du métier.

L'intérêt d'une telle configuration réside essentiellement dans la possibilité d'introduire dans l'insert de l'invention un élément que l'on ne peut introduire dans le métier, ou que l'on ne peut insérer dans le tube concerné après réalisation de la structure textile. Un tel élément est ainsi associé à l'insert de l'invention par confection, et notamment couture des deux bords libres (207) du tube concerné autour dudit élément. Un tel élément peut notamment être constitué de l'anneau d'une valve cardiaque, et de manière générale de tout élément nécessaire à la fonctionnalisation de l'insert.

Les figures 3 et 4 illustrent un second mode de réalisation. Selon ce mode de réalisation, on réalise lors de la fabrication de l'insert sur le métier, des ailettes (303) et (403). Ces ailettes résultent en fait du liage sur le métier des tubes connexes au tube principal (302, 402). Ces parties plates ou ailettes (303, 403) permettent ainsi de faciliter d'une part la formation de certaines liaisons à l'intérieur de l'organisme, favorisant ainsi la colonisation, et d'autre part l'intervention du praticien, la fixation de l'implant étant plus aisée.

Les figures 5 et 6 représentent, comme déjà dit, des variantes de réalisation des tubes illustrés dans les figures précédentes. Les tubes (501) ou (601), parallèles entre eux et orientés sens production, ne sont plus disposés de manière tangentielle les uns aux autres, mais sont reliés entre eux par des parties plates (502) et (602), résultant du liage sur le métier. La largeur de ces zones plates (502, 602) dépend de l'importance de ce liage.

La figure 7 représente un tricot obtenu par la technologie maille jetée conforme à la présente invention. Sur cette figure a été représenté schématiquement une structure de fond (701) correspondant au premier modèle de multi-micro tube présenté en figures 1 et 2. On peut également observer le travail des fils sur chaque fonture (702) et (703), travail visant à créer un tube principal, avec en bordure, le travail des fils passant d'une fonture à l'autre afin d'emprisonner les tiges et éléments rapportés (704), tels que par exemple des fils métalliques (par exemple en cuivre, en Nickel, en Argent, en Or, en Aluminium) ou à mémoire de forme de type Nitinol ou en polymère (par exemple PET (polyéthertéréphtalate, polypropylène, polyéthylène, polytétrafluoroethylène, e-PTFE, PLA, PGA, PCL, PDO, aramides.

Les fils constituant le tricot peuvent être mono ou multi-filaments et sont choisis dans une large gamme de matériaux polymères naturels ou synthétiques, résorbables ou non.

Cette technologie présente par ailleurs l'avantage de jouer avec le jeu des fils, mais aussi avec la densité de ceux-ci dans les sens production et sens travers, déterminant de ce fait la porosité des multi-micros tubes, facilitant donc le greffage de l'implant dans l'organisme.

La figure 8 représente également un tricot obtenu par maille jetée conforme à l'invention. Elle se rapporte à la structure correspondant aux figures 3 et 4, c'est-à-dire au modèle de micro-tube avec partie plate adjacente. La même technique de liage est utilisée pour réaliser le tube : celui-ci est réalisé sur double fonture tandis qu'un autre liage est utilisé pour obtenir la partie plate. On combine alors des liages avec simple tricot (801) et satin (802). Ceux-ci représentent des trames partielles ou flottées propices à l'accrochage dans l'organisme.

La finition de l'insert, notamment au niveau de ses bords latéraux se doit d'être la plus fine possible, et avantageusement exempte d'extrémités libres de fils, ce afin de ne représenter aucun risque de danger pour l'organisme lors de l'introduction. Cette finition est avantageusement réalisée par l'ajout de chaînettes (803) en lisière du produit ce afin d'augmenter la précision la construction textile avec liage.

Lesdites bordures peuvent en outre recevoir par un maillage approprié des fils insérés (804), tel que par exemple réalisés en métal, en alliage métallique (Nitinol), ou en polymère, à l'instar des fils (704) de la figure 7.

## Revendications

1. Insert textile tricoté en trois dimensions obtenu en une seule étape par technologie à maille jetée sur métier Rachel à double fonture ou sur métier crochet, comportant en sens production au moins deux tubes (102, 202 ; 103, 203 ; 501 ; 601) s'étendant parallèlement entre eux, et séparés entre eux par une zone de liage (104, 204) au niveau de laquelle les nappes résultant du tricotage sont liées entre elles, respectivement un tube principal (102, 202) de diamètre le plus important et au moins un tube connexe (103, 203), ***caractérisé* en ce que** l'on introduit dans un ou les tubes connexes, c'est-à-dire autres que le tube dit principal, en une seule étape lors de la réalisation de l'insert sur le métier à tricoter, un fil droit ou un assemblage de fil montés en cabestan (206), qui ne maille pas, une bandelette, des fibres optiques, ou tout autre matériau dont la dimension principale est la longueur, une tige, un jonc, ou un élément rigidifiant apte à faciliter la mise en œuvre et l'implantation de l'insert.

2. Insert textile tricoté en trois dimensions selon la revendication 1, ***caractérisé* en ce que** les tubes (102, 202 ; 103, 203 ; 501 ; 601) sont tangents entre eux ou séparés l'un de l'autre ou les uns des autres par une zone plane (502, 602).

3. Insert textile tricoté en trois dimensions selon l'une des revendications 1 et 2, ***caractérisé* en ce que** les tubes (102, 202 ; 103, 203 ; 501 ; 601) qu'il comporte sont de diamètres différents.

4. Insert textile tricoté en trois dimensions selon la revendication 3, ***caractérisé* en ce que** le tube de diamètre le plus important, dit tube principal (102, 202), reçoit un cathéter, un tube en Nitinol ou un élément implantable (205).

5. Insert textile tricoté en trois dimensions selon l'une des revendications 1 à 4, ***caractérisé* en ce que** la porosité de l'insert est définie par les liages ou armures mises en œuvre sur le métier, ladite porosité pouvant être différente d'un tube à l'autre de l'insert.

6. Insert textile tricoté en trois dimensions selon la revendication 5, ***caractérisé* en ce que** l'ouverture des pores de l'insert, définie par les liages ou armures est comprise entre 0,05 et 3 millimètres dans leur plus grande dimension.

7. Insert textile tricoté en trois dimensions selon l'une des revendications 1 à 6, ***caractérisé* en ce que** les fils qui le constituent sont des fils mono-filaments ou multi-filaments.

8. Insert textile tricoté en trois dimensions selon l'une des revendications 1 à 7, ***caractérisé* en ce que** l'un des tubes est ouvert (207) lors du tricotage.

9. Insert textile tricoté en trois dimensions selon l'une des revendications 1 à 8, ***caractérisé* en ce que** les fils qui le constituent sont réalisés en polymère ou copolymère synthétique, résorbable ou non.

10. Insert textile tricoté en trois dimensions selon l'une des revendications 1 à 8, ***caractérisé* en ce que** les fils qui le constituent sont réalisés en polymère d'origine naturelle, résorbable ou non.

11. Insert textile tricoté en trois dimensions selon l'une des revendications 1 à 10, ***caractérisé* en ce que** d'autres fils sont introduits dans l'insert lors de la réalisation de ce dernier, afin de lui conférer des propriétés particulières, ces fils insérés (704, 804) étant métalliques, ou à mémoire de forme de type Nitinol ou en polymère.

## Patentansprüche

1. Dreidimensional gestrickte Textileinlage, entstanden in einem einzigen Schritt durch Kettenwirktechnik, auf einer Doppelfontur-Rachelmaschine oder einer Häkelmaschine, diese enthält in Produktionsrichtung mindestens zwei Schläuche (102, 202 ; 103, 203 ; 501 ; 601), zueinander parallel verlaufend und getrennt durch eine Strukturzone (104, 204) in deren Höhe die Bahnen aus dem Wirkvorgang miteinander verbunden sind, jeweils ein Hauptschlauch (102, 202) mit einem größeren Durchmesser und mindestens ein Nebenschlauch (103, 203), ***dadurch gekennzeichnet, dass*** in den oder die Nebenschläuche, also die nicht den sogenannten Hauptschlauch bilden, in einem einzigen Schritt bei der Herstellung der Einlage auf der Strickmaschine ein gerader Faden oder eine Verbindung verwundener Fäden (206), die also keine Maschen bilden, ein Band, optische Fasern oder jedes andere Material eingeführt wird, deren wichtigste Dimension die Länge ist, eine Stange, ein Ring oder ein Aussteifungselement, deren Zweck darin besteht, das Umsetzen der Implantation der Einlage zu erleichtern.

2. Dreidimensional gestrickte Textileinlage nach Anspruch 1, ***dadurch gekennzeichnet, dass*** die Schläuche (102, 202, 103, 203, 501, 601) einander tangieren oder voneinander durch eine flache Zone (502, 602) getrennt sind.

3. Dreidimensional gestrickte Textileinlage nach einem der Ansprüche 1 und 2, ***dadurch gekennzeichnet, dass*** die Schläuche (102, 202, 103, 203, 501, 601) aus denen sie besteht, unterschiedliche Durchmesser haben.

4. Dreidimensional gestrickte Textileinlage nach Anspruch 3, ***dadurch gekennzeichnet, dass*** der Schlauch mit dem größten Durchmesser, der sogenannte Hauptschlauch (102, 202), einen Katheter, ein Nitinol- Röhrchen oder ein implantierbares Element (205) aufnimmt.

5. Dreidimensional gestrickte Textileinlage nach einem der Ansprüche 1 bis 4, ***dadurch gekennzeichnet, dass*** die Porosität der Einlage durch die auf der Maschine eingesetzten Strukturen oder Bindungen definiert wird, wobei sich diese Porosität zwischen den einzelnen Schläuchen der Einlage unterscheiden kann.

6. Dreidimensional gestrickte Textileinlage nach Anspruch 5, ***dadurch gekennzeichnet, dass*** die Öffnung der Poren der Einlage, definiert durch die Strukturen oder Bindungen zwischen 0,05 und 3 Millimetern in der größten Dimension liegt.

7. Dreidimensional gestrickte Textileinlage nach einem der Ansprüche 1 bis 6, ***dadurch gekennzeichnet, dass*** die Fäden, aus denen sie besteht, Monofilament- oder Multifilamentfäden sind.

8. Dreidimensional gestrickte Textileinlage nach einem der Ansprüche 1 bis 7, ***dadurch gekennzeichnet, dass*** einer der Schläuche beim Stricken offen (207) ist.

9. Dreidimensional gestrickte Textileinlage nach einem der Ansprüche 1 bis 8, ***dadurch gekennzeichnet, dass*** die Fäden, aus denen sie besteht, aus synthetischem Polymer oder Copolymer, resorbierbar oder nicht, hergestellt sind.

10. Dreidimensional gestrickte Textileinlage nach einem der Ansprüche 1 bis 8, ***dadurch gekennzeichnet, dass*** die Fäden, aus denen sie besteht, aus Polymer natürlicher Herkunft, resorbierbar oder nicht, hergestellt sind.

11. Dreidimensional gestrickte Textileinlage nach einem der Ansprüche 1 bis 10, ***dadurch gekennzeichnet, dass*** andere Fäden während der Herstellung in die Einlage eingeführt werden, um ihr besondere Eigenschaften zu verleihen, diese eingelegten Fäden (704, 804) können aus Metall oder aus Formgedächtnisdraht vom Typ Nitinol oder aus Polymer bestehen.

## Claims

1. A three-dimensional knitted textile insert obtained in a single step by warp knitting technology, on a double-needle Raschel machine or on a crocheting machine, comprising in the production direction at least two tubes (102, 202; 103, 203; 501 ; 601) extending parallel to one another, and separated from one another by a binding area (104, 204) at the level of which the sheets resulting from the knitting are interlinked, respectively a main tube (102, 202) of greater diameter and at least one side tube (103, 203), ***characterized* in that** a straight yarn or an assembly of capstan-mounted yarns (206), which thus does not loop, a band, optical fibers, or any other material having length as a main dimension, a rod, a cord, or a rigidifying element, intended to ease the implementation of the insert implantation, is introduced into one or the side tubes, that is, other than the so-called main tube, in a single step on forming of the insert on the knitting machine.

2. The three-dimensional knitted textile insert according to claim 1, ***characterized* in that** the tubes (102, 202; 103, 203; 501; 601) are tangent to one another or separated from each other or from one another by a planar area (502, 602).

3. The three-dimensional knitted textile insert according to any of claims 1 and 2, ***characterized* in that** the tubes (102, 202; 103, 203; 501; 601) that it comprises have different diameters.

4. The three-dimensional knitted textile insert according to claim 3, ***characterized* in that** the tube having the largest diameter, called main tube (102, 202) receives a catheter, a Nitinol tube, or an implantable element (205).

5. The three-dimensional knitted textile insert according to any of claims 1 to 4, ***characterized* in that** the porosity of the insert is defined by the bindings or weaves implemented on the machine, and said porosity may be different from one tube to the other of the insert.

6. The three-dimensional knitted textile insert according to claim 5, ***characterized* in that** the opening of the insert pores, defined by the bindings or weaves, is in the range from 0.05 to 3 millimeters in their largest dimension.

7. The three-dimensional knitted textile insert according to any of claims 1 to 6, ***characterized* in that** the yarns are monofilament or multifilament yarns.

8. The three-dimensional knitted textile insert according to any of claims 1 to 7, ***characterized* in that** one of the tubes is open (207) during the knitting.

9. The three-dimensional knitted textile insert according to any of claims 1 to 8, ***characterized* in that** the yarns forming it are made of synthetic polymer or copolymer, resorptive or not.

10. The three-dimensional knitted textile insert according to any of claims 1 to 8, ***characterized* in that** the yarns forming it are made of polymer of natural origin, resorptive or not.

11. The three-dimensional knitted textile insert according to any of claims 1 to 10, ***characterized* in that** other yarns are introduced into the insert on forming thereof, to give it specific properties, the inserted yarns (704, 804) being metallic or shape-memory yarns of Nitinol type, or made of polymer.
